(19) Europäisches Patentamt
European Patent Office
Office européen des brevets

(11) Publication number: 0 017 938
B1

(12) **EUROPEAN PATENT SPECIFICATION**

(45) Date of publication of patent specification: 03.08.83

(51) Int. Cl.³: C 12 P 21/02, C 07 C 103/52

(21) Application number: 80101966.2

(22) Date of filing: 11.04.80

(54) **Process for preparing a B30-threonine insulin.**

(30) Priority: 13.04.79 JP 45710/79
13.04.79 JP 45711/79

(43) Date of publication of application:
29.10.80 Bulletin 80/22

(45) Publication of the grant of the patent:
03.08.83 Bulletin 83/31

(84) Designated Contracting States:
CH DE FR GB IT LI NL

(56) References cited:
CH - A - 599 927
CH - A - 602 599
FR - A - 2 100 978
GB - A - 1 465 235
US - A - 3 276 961
US - A - 4 116 768

NATURE, vol. 280, no. 5721, August 2, 1979, Macmillan Journals K. MORIHARA et al.: "Semi-synthesis of human insulin by trypsincatalysed replacement of Ala-B30 by Thr in porcine insulin" pages 412—413

(73) Proprietor: SHIONOGI & CO., LTD.
12, Dosho-machi 3-chome Higashi-ku
Osaka 541 (JP)

(72) Inventor: Morihara, Kazuyuki
5-9-2, Hirose, Shimamoto-cho
Mishima-gun, Osaka Pref (JP)
Inventor: Oka, Tatsushi
150-21, Shinpukuji, Mihara-cho
Minamikawachi-gun, Osaka Pref (JP)
Inventor: Tsuzuki, Hiroshige
1-4-307, Minami-machi, Murano
Hirakata-shi, Osaka Pref (JP)
Inventor: Soejima, Masami
847-1, Komatsu-cho
Tsuchiura-shi, Ibaraki Pref (JP)
Inventor: Masaki, Takeharu
3998, Ami-cho
Inashiki-gun, Ibaraki Pref (JP)

(74) Representative: Vossius-Vossius-Tauchner-Heunemann-Rauh Patentanwälte
P.O.Box 860767 Siebertstrasse 4
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 017 938 B1

# Process for preparing a B30-threonine-insulin

This invention relates to an enzymatic process for preparing a B30-threonine-insulin, e.g. human insulin, and to novel B30-threonine-insulins where the OH and/or COOH groups are protected.

Insulin is indispensable for the treatment of diabetes mellitus. Bovine and porcine insulin are commercially available at present. Human insulin is different from other animal insulins in the amino acid residues in positions 8, 9, and 10 of the A chain and in position 30 of the B chain. The porcine insulin is most similar to that of human insulin and differs only by the substitution of an alanine residue at position 30 of the B chain. These amino acid residues cause formation of antibodies in patients. These antibodies are known to decrease the effectiveness of further insulin treatment. Therefore, the treatment of diabetes with human insulin is to be preferred.

Human insulin has been prepared by chemical methods; see U.S. Patent No. 3,903,068 and R. Obermeier and R. Geiger, Hoppe-Seyler's Z. Physiol. Chem., 357 (1976), 759—767. These processes comprise the condensation of a desoctapeptide-(B23—30)-porcine insulin with a synthetic octapeptide corresponding to position B23—30 of human insulin. The first mentioned process includes an alkaline hydrolysis which causes adverse side-reactions. The latter process is also a nonspecific reaction with various side-reactions and requires complicated purification procedures. Therefore, these processes cannot be applied on an industrial scale.

M. Bodanszky et al. describe a process for preparing human insulin in U.S. Patent No. 3,276,961 wherein an animal insulin containing a C-terminal alanine group in the B-chain and a group consisting of asparagine and aspartic acid as the C-terminal group of the A-chain is subjected to the action of carboxypeptidase or trypsin in the presence of threonine. This process is not likely to produce human insulin because trypsin and carboxypeptidase A hydrolyze not only the peptide bond of lysyl-alanine (B29—B30) but also other positions in insulin under the conditions described there. Trypsin hydrolyzes the peptide bond of arginyl-glycine (B22—B23) faster than that of lysyl-alanine (B29—B30). Carboxylpeptidase A cannot specifically remove the C-terminal alanine group in the B-chain without splitting off the C-terminal asparagine group in the A-chain. Special reaction conditions, i.e. working in an ammonium hydrogencarbonate buffer solution, are necessary to prevent the removal of the asparagine group. These reaction conditions are described in Hoppe-Seyler's Z. Physiol. Chem., 359 (1978), 799—802. Furthermore, a peptide synthesis can hardly occur because the hydrolysis rate is faster than the synthesis rate under the conditions described.

Until now there is no process for preparing a B30-threonine-insulin known which is useful on a commercial scale.

It is the object of this invention to provide a B30-threonine-insulin, e.g. human insulin, in commercially feasible quantities by an enzymatic process starting from a des-B30-insulin and a L-threonine derivative. It is another object of this invention to provide a new process for preparing a B30-threonine-insulin from des-B30-insulin. Finally it is an object of this invention to provide novel intermediates for preparing the B30-threonine-insulines. These objects are accomplished by the process of this invention which essentially comprises

a) reacting a L-threonine derivative of the general formula I

$$Thr(R^1)(R^2) \qquad (I)$$

wherein Thr is the L-threonine group, $R^1$ denotes a hydrogen atom or a hydroxyl protecting group and $R^2$ is a carboxyl protecting group, with a des-B30-insulin in a molar ratio of 5 : 1 to 1000 : 1 in the presence of an enzyme specifically acting on the basic amino acid carbonyl groups in peptide linkages and

b) is desired, removing the protecting group(s) from the B30-Thr($R^1$)($R^2$)-insulin obtained.

The process of this invention is rather specific and is not accompanied by any adverse side reactions such as racemization. Additionally, unreacted starting materials can be easily recovered and reutilized. The process of this invention uses an excess amount of the L-threonine derivative (I). The excess amount of the L-threonine derivative (I) prevents the cleavage of the peptide linkage at the carbonyl group contributed by the arginine group at 22 position of the B-chain, although this CO-NH-bond is usually split by trypsin. Accordingly, a protection of the arginine group which is indispensable in conventional enzymatic reactions with insulin is not necessary in this process.

Des-B30-insulin can be obtained by reacting various animal insulins obtained from the pancreas glands of e.g. pigs, cattle, whales, sheep, rabbits, fish, monkey and others with carboxypeptidase A as disclosed by E. W. Schmitt et al. Hoppe-Seyler's Z. Physiol. Chem., 359 (1978, 799, Des-B30-insulin may also be prepared by using a protease produced by Achromobacter lyticus. This protease specifically cleaves the peptide linkage at the carbonyl group contributed by lysine. The isolation and characteristics of this enzyme are described by Masaki et al. in Agric. Biol. Chem., 42 (1978), 1443. The enzyme is called Protease I. The preparation of des-B30-porcine insulin is disclosed in Biochem. Biophys. Res.

Commun., 92 (1980), 396—402 and Japanese Patent Publication (not-examined) 135789/1979. Des-B30-insulins of other animals can be prepared in the same manner.

The hydroxy group of the threonine derivative of the general formula I may be protected or free. Both compounds can be used in the process of this invention. The protecting group may be any hydroxy-protecting group generally used in peptide synthesis, e.g. a tert.-butyl, benzyl or acetyl group. The carboxyl group of the threonine derivative of the general formula I has to be protected and any carboxyl-protecting group utilized in peptide synthesis can be employed. Typical protecting groups are, for example, alkyl esters, e.g. the tert.-butyl ester, aralkyl esters, e.g. the benzyl ester, amides, substituted amides, e.g. the anilide, and salts, e.g. the sodium salt.

The protecting group should be chosen considering the influence of the introduction and removal reactions to the insulin because these reactions might result in a denaturation or inactivation of the insulin. If the hydroxy-protecting group and the carboxyl-protecting group for the L-threonine are properly selected, a single cleavage procedure is sufficient to remove both protecting groups. Protecting groups used in peptide synthesis are described e.g. by M. Bodanszky et al., Peptide Synthesis, second edition (1976) published by John Wiley & Sons.

The enzyme used in the process of this invention is specifically cleaving peptide linkages in a substrate protein whose carbonyl groups are contributed by basic amino acids. Such enzymes may be obtained from various plants and animals. Examples are trypsin and trypsin-like enzymes. Examples of trypsin-like enzymes are the protease isolated by Morihara et al., Arch. Biochem. Biophys., 126 (1968), 971, and the protease disclosed by Yoshida et al., FEBS Lett., 15 (1971), 129. The enzyme to be used may be treated with tosyl-L-phenylalanine chloromethylketone to remove admixed enzymes such as chymotrypsin and chymotrypsin-like enzymes.

Another trypsin-like enzyme is protease I produced by Achromobacter lyticus M497—1. As stated above protease I cleaves specifically the peptide linkages in a substrate protein whose carbonyl groups are contributed by lysine.

The condensation of the L-threonine derivative (I) with the des-B30-insulin is effected under suitable conditions for the peptide synthesis of the above enzymes. The reaction is preferably carried out at a pH of 5 to 9, preferably at a pH of 6 to 7. The reaction temperature is from 0 to 50°C, preferably from 20 to 40°C. It is desired that the concentrations of the L-threonine derivative and the des-B30-insulin are as high as possible. The molar ratio of the L-threonine derivative to des-B30-insulin is 5 : 1 to 1000 : 1, preferably 25 : 1 to 200 : 1.

Water-miscible organic solvents are preferably added to the reaction mixture. The addition of the organic solovent remarkably increases the solubility of the starting compounds and lowers the water concentration of the reaction mixture resulting in supression of the reverse reaction, i.e. the hydrolysis of the product. Examples of the organic solvents to be used are methanol, ethanol, dimethylformamide, dimethyl sulfoxide and glycerol. These solvents can be used either individually or as a mixture. The preferred concentration of the organic solvent is about 0 to 65%, especially 40 to 60% of the reaction mixture. The amount of the organic solvent depends on the solubility of the starting materials, the tendency of the enzyme to denature and its hydrolyzing activity.

Examples of useful reaction media are trishydroxymethylaminomethane (abbreviated tris hereinafter), carbonate and borate buffer solutions. The enzyme concentration depends on the concentration of the starting materials and the enzyme activity. For example, crystalline trypsin is used preferably in a concentration from about 1 mg/ml to 10 mg/ml. The enzyme may be used as such or in a fixed form e.g. combined with or included in an insoluble carrier such as cellulose, a dextran (e.g. Sephadex (trade mark)), agarose (e.g. Sepharose (trade mark)), a polyacrylamide gel (Bio-gel (trade mark)), or porous glass.

The reaction time varies and is affected by other reaction conditions. The reaction is generally continued until an equilibrium is reached between the starting materials and the product. The reaction time is generally about 3 to 72 hours and in most cases about 6 to 24 hours.

The B30-Thr($R^1$)($R^2$)-insulin obtained is isolated according to conventinal methods used in peptide chemistry. One example for an isolation procedure is as follows: The reaction mixture is subjected to a gel filtration to isolate the unreacted L-threonine derivative and the enzyme which can be reutilized. The remaining part is subjected to chromatography to isolate the B30-Thr($R^1$)($R^2$)-insulin and unreacted des-B30-insulin. The latter can be reused.

From the B30-Thr($R^1$)($R^2$)-insulin the protecting group(s) may be removed. The removal of the protecting group(s) is effected in the usual manner, through the specific method will depend on the properties of the protecting group(s). The tert.-butyl group is an example of a protecting group for both the hydroxy and carboxyl group of L-threonine. It may be removed by treating the B30-Thr($R^1$)($R^2$)-insulin wherein $R^1$ and $R^2$ are tert.-butyl groups with trifluoroacetic acid in the presence of a cation-trapping agent, e.g. anisole. As noted above, a single treatment may remove the hydroxy-protecting and the carboxyl-protecting groups simultaneously. Thus the process becomes simple and the B30-threonine insulin is obtained in good yield. When the C-terminal of

the resulting insulin is protected in another ester, amide, substituted amide or salt form the protecting group may be removed by a suitable hydrolysis or desalting technique.

The B30-threonine insulin prepared according to the process of this invention, in particular if prepared from porcine des-B30-insulin, in useful for the treatment of diabetes mellitus and also as a reagent. The insulin obtained by this invention shows the same activity of lowering blood glucose in mice as bovine insulin. The test is carried out as follows:

The test compound is dissolved in 0.01 M hydrochloric acid and the solution is diluted to a suitable concentration (2.5—20 μg/ml) with 20 to 60 times of physiological salt solution. The resulting solution is intravenously administered to DS mice fasted for 5 hours in a dose of 0.1 ml/10 g body weight. Blood is taken from the orbital blood vessel (i.e. the eye socket sinus) 45 minutes after the administration and blood glucose is measured using the glucose-oxidase method.The results are shown in Table I.

TABLE I

Insulin Activity

| Test Compound | Dose (μg / 10g Body Weight) | Blood Glucose Level (mg / dl) |
|---|---|---|
| Physiological Salt Solution | – | 112.2 ± 7.3 (1) |
| Semi-synthetic Human Insulin | 0.25<br>1 | 80.2 ± 2.6 (2)<br>41.0 ± 6.0 (3) |
| Bovine Insulin | 0.25<br>1 | 73.2 ± 1.5 (4)<br>41.0 ± 2.3 (5) |

Note: (2) – (4), (3) – (5) No significant difference.

Human insulin prepared by this invention from porcine des-B30-insulin can be administered to man in the same manner as porcine or bovine insulin preparations. It may be converted to conventional pharmaceutical insulin preparations. For example, it may be converted into an injectable solution by a suitable procedure such as those for preparing a zinc complex with zinc chloride, a buffered solution with a suitable buffer such as sodium hydrogenphosphate, sodium acetate and the like, or an isotonic solution with sodium chloride.Furthermore, suitable preservatives may be added to the insulin preparation for example cresol, phenol or a para-hydroxybenzoic acid alkyl ester, e.g. the methyl, ethyl, propyl or butyl ester. The dosage of the human insulin is the same as for conventional insulin preparations. About 1 to 100 units may be administered to a human adult per day, though the dosage depends on the severity of diabetes.

The following examples are given to further illustrate the present invention. The abbreviations have the following meaning: Ala: alanine, Arg: arginine, Asp: aspartic acid, $CysO_3H$: cysteic acid, Glu: glutamic acid, Gly: glycine, His: histidine, Ile: isoleucine, Leu: leucine, Lys: lysine, Phe: phenylalanine, Pro: proline, Ser: serine, Thr: threonine, Tyr: tyrosine, Val: valine, $OBu^t$: tert.-butyl ester residue, mM: mmoles per ml. TPCK: tosyl-L-phenyl-alanine chloromethylketone.

Example 1

(1) Desalanine-(B30)-porcine insulin

To a solution of porcine insulin (500 mg) in 0,1 m ammonium hydrogencarbonate (100 ml, pH 8.3) is added crystalline carboxypeptidase A (5 mg, Worthington Co., pretreated with diisopropylfluorophasphate, 49 u/mg). The mixture is incubated at room temperature for 8 hours. The incubation is stopped when alanine is released at a ratio of 0.77 M/1M insulin. The reaction mixture is lyophilized. The product is dissolved in 0.5 M acetic acid and applied to a column (3.5 x 95 cm) filled with super fine particles of Sephadex G 50. The column is eluted with 0.5 M acetic acid. Fractions of 11.5 ml are collected. Fractions nos. 40—60 are pooled and lyophilized to give the title compound (460 mg). The yield is 92%.

The product is hydrolyzed for the amino acid analysis with 6 M hydrochloric acid at 110°C for 24 hours to give the following result (theoretical values in paranthesis): Lys 1.00 (1), His 1.91 (2), Arg 0.95 (1), Asp 3.21 (3), Thr

2.09 (2), Ser 2.97 (3), Glu 7.35 (7), Gly 4.29 (4), Ala 1.26 (1), $CysO_3H$ 5.94 (6), Val 3.86 (4), Ile 1.55 (2), Leu 6.53 (6), Tyr 4.08 (4), Phe 3.22 (3), Pro 1.2 (1).

(2) [B30-Thr-$OBu^t$]-porcine insulin

The above product (100 mg, 10 mM) and L-threonine tert.-abutyl ester (154 mg, 500 mM) are dissolved in a mixture (1.05 ml) of ethanol and dimethylformamide (1 : 1, v/v). To the solution is added a solution of crystalline trypsin (4 mg, Worthington Co., recrystallized three times) in 0.5 M borate buffer solution (0.75 ml, pH 6.5) and TPCK in a final concentration of 0.01 mM. The mixture is kept overnight at 37°C. The product is confirmed by high pressure liquid chromatography and the calculated yield is 75%. The mixture is acidified with glacial acetic acid and subjected to gel filtration using a 4.2 x 130 cm column filled with super fine particles of Sephades G 50 to give three fractions corresponding to trypsin, the desired insulin derivative and L-threonine tert.-butyl ester. Measurement of the enzyme activity and the Ninhydrin reaction reveal that trypsin and L-threonine tert.-butyl ester are each recovered in a yield of 50%.

The fraction containing the desired insulin derivative is lyophilized to yield a crude powder (89 mg) of the product. The product is applied at 4°C to a 1.9 x 24.5 cm column of DEAE-Sephades A 25 which has been previously equilibrated with 0.01 M tris buffer solution (pH 7.6) and 7 M urea. The column is eluted with the above buffer solution (800 ml) and then with a linear $Na^+$ gradient (to 0.3 M NaCl) to give a fraction A near to 0.08 to 0.09 M concentration and a fraction B near to 0.13 to 0.14 M. Each fraction is immediately dialyzed against 0.01 M ammonium acetate for 3 to 4 days under cooling and lyophilized to give a powder (35 mg) from the fraction A and a powder (27 mg) from the fraction B. The former is identified to be the title compound and the latter is identified to be a mixture of desalinine-(B30)-porcine insulin and intact porcine insulin by high pressure liquid chromatography and polyacrylamide gel electrophoresis.

(3) Human insulin

A mixture of trifluoroacetic acid (2 ml) and anisole (0.2 ml) is added to 30 mg [B30-Thr-$OBu^t$]-porcine insulin obtained above and the mixture is kept at room temperature for 30 minutes. The trifluoroacetic acid is removed under a nitrogen atmosphere and the mixture is extracted with ether (15 ml) after addition of 1N acetic acid (2 ml) to remove the anisole. The acetic acid layer is lyophilized to give 26 mg of the title compound. The yield is 43% based on the starting material having a purity of 77%.

The product, i.e. the semi-synthetic human insulin is identified to be the title compound by amino acid analysis, Slab gel electrophoresis and high pressure liquid chromatography. The amino acid analysis is performed under the same conditions as in (1) and the following results is obtained: Lys 1.00 (1), His 1.89 (2), Arg 0.87 (1), Asp 3.32 (3), Thr 3.16 (3), Ser 2.99 (3), Glu 7.35 (7), Pro 1.29 (1), Gly 4.39 (4), Ala 1.26 (1), $CysO_3H$ 4.6 (6), Val 3.93 (4), Ile 161 (2), Leu 6.51 (6), Tyr 3.68 (4), Phe 3.19 (3).

## Example 2

(1) Desalanine-(B30)-bovine insulin

To a solution of bovine insulin (200 mg) in 0.1 M ammonium hydrogencarbonate (40 ml) is added crystalline carboxypeptidase A (1.8 mg, 49 μ/mg). The mixture is kept overnight at room temperature and then lyophilized to give 180 mg of a crude powder consisting of 78% of the title compound and 22% of desasparagine-(A21)-desalanine-(B30)-insulin.

(2) [B30-Thr-$OBu^t$]-bovine insulin

The powder (116 mg, 5 mM) obtained above (1) and L-threonine tert.-butyl ester (360 mg, 500 mM) are dissolved in a mixture (2.4 ml) of ethanol and dimethylformamide (1 : 1). The solution is mixed with 0.5 M borate buffer solution (1.6 ml, pH 7.0) containing crystalline trypsin (87.5 Mg, 0.94 mM) and TPCK (0.27 mg, 0.2 mM) and incubated overnight at 37°C. The formation of the title compound is confirmed by high pressure liquid chromatography and the yield is calculated to be 80%.

The reaction mixture is treated in the same manner as in Example 1 (2). The resulting fraction A is again chromatographed to remove desasparagine-(A21)-desalanine-(B30) - insulin and treated in the same manner as in Example 1 (2) to give 63 mg of a crude powder. The product is identified to be the title compound by high pressure liquid chromatography and Slab gel electrophoresis.

(3) [B30-Thr]-bovine insulin

To the product (63 mg) obtained above is added a mixture of trifluoroacetic acid (2 ml) and anisole (0.2 ml). The mixture is kept at room temperature for 30 minutes, then treated in the same manner as in Example 1 (3) and lyophilized to give 60 mg of the title compound. The yield is 66% based on the starting compound having a purity of 78%.

The product is compared with authentic [B30-Thr]-bovine insulin prepared by another method by amino acid analysis, Slab gel electrophoresis and high pressure liquid chromatography and is found to be identical. The amino acid analysis is performed under the same conditions as in Example 1 (1) and the following result is obtained: Lys 1.00 (1), His 1.95 (2), Arg 0.98 (1), Asp 3.00 (3), Thr 2.00 (2), Ser 2.83 (3), Glu 6.69 (7), Gly 4.06 (4), Ala 2.06 (2), $CysO_3H$ 5.38 (6), Val 4.45 (5), Ile 0.76 (1), Leu 6.14 (6), Tyr 3.92 (4), Phe 2.97 (3), Pro 1.20 (1).

Example 3

(1) [B30-Thr-$OBu^t$]-porcine insulin

Desalanine-(B30)-porcine insulin (100 mg, 10 mM) prepared according to the process of Example 1 (1) and L-threonine tert.-butyl ester (205 mg, 500 mM) are dissolved in a mixture (1.05 ml) of ethanol and dimethylformamide (1 : 1, v/v). The solution is mixed with a 0.5 M borate buffer solution (0.7 ml) of protease I (0.35 mg) produced by Achromobacter lyticus M497—1 and incubated at 37°C for 2 days. The reaction mixture is acidified with glacial acetic acid and subjected to gel filtration using a 4.2 x 130 cm column filled with super fine particles of Sephadex G 50. An enzyme fraction, an insulin fraction and a L-threonine tert.-butylester fraction is obtained. The enzyme and the L-threonine tert.-butyl ester are recovered in a yield of more than 50%. The insulin fraction is lyophilized to give a crude powder (83 mg). The powder is subjected to column chromatography in the same manner as in Example 1 (2) to give a powder (48 mg) from the fraction A and another powder (20 mg) from the fraction B. The former is identified to be the title compound and the latter is identified to be a mixture of desalanine-(B30)-porcine insulin and porcine insulin.

(2) Human insulin

40 mg [B30-Thr-$Obu^t$]-porcine insulin obtained above is treated with trifluoroacetic acid (2 ml) and anisole (0.2 ml). The mixture is kept at room temperature for 30 minutes and then treated in the same manner as in Example 1 (3) to give 37 mg of the title compound. The yield is 56% based on the starting compound having a purity of 76%.

The product is identified to be the title compound by amino acid analysis, Slab gel electrophoresis and high pressure liquid chromatography. The amino acid analysis is performed under the same conditions as in Example 1 (1) and the following result is obtained: Lys 1.00 (1), His 1.90 (2), Arg 0.93 (1), Asp 3.21 (3), Thr 3.05 (3), Ser 2.99 (3), Glu 7.23 (7), Pro 1.25 (1), Gly 4.33 (4), Ala 1.25 (1), Val 3.91 (4), Ile 1.58 (2), Leu 6.51 (6), Tyr 3.66 (4), Phe 3.15 (3).

Example 4

(1) [B30-Thr($OBu^t$)-$OBu^t$]-porcine insulin

Desalanine-(B30)-porcine insulin (100 mg, 10 mM) prepared according to the process of Example 1 (1) and O-tert.-butyl-threonine tert.-butyl ester (203 $\mu$l, 500 mM) are dissolved in a mixture of ethanol (325 $\mu$l) and dimethylformamide (352 $\mu$l). The solution is mixed with a 0.5 m borate buffer solution (0.75 ml) of protease I (26 $\mu$l, 30 $\mu$M) produced by Achromobacter lyticus M497-A. The final pH of the mixture is 6.7. The mixture is incubated at 37°C for 4 hours. The formation of [B30-Thr($OBu^t$)-$OBu^t$]-porcine insulin is confirmed by high pressure liquid chromatography and the calculated yield is 84%. After the incubation the reaction mixture is treated in the same manner as in example 1 (2) to give a powder (62 mg) of [B30-Thr($OBu^t$)-$OBu^t$]-porcine insulin and a powder (27 mg) of desalanine-(B30)-porcine insulin.

(2) Human insulin

The [B30-Thr($OBu^t$)-$OBu^t$]-porcine insulin powder is treated with trifluoroacetic acid and anisole in the same manner as in example 1 (3) to give human insulin (60 mg).

Example 5

(1) The reaction is effected in the same manner as in example 4 (1) under the following conditions:

| | |
|---|---|
| Desalanine-(B30)-procine insulin | 116 mg (5 mM) |
| O-tert.-butyl-threonine tert.-butyl ester | 472 $\mu$l (500 mM) |
| 0.5 M borate buffer | 1500 $\mu$l |
| Ethanol | 780 $\mu$l |
| Dimethylformamide | 780 $\mu$l |
| Achromobacter protease (2 mM) | 61 $\mu$l (30 $\mu$M) |

The production of [B30-Thr($OBu^t$)-$OBu^t$]-porcine insulin is confirmed by high pressure liquid chromatography and the calculated yield is 70%. After the incubation the reaction mixture is heated in the same manner as in example 1 (2) to give [B30-Thr($OBu^t$)-$OBu^t$]-porcine insulin (64 mg) and desalanine-(B30)-porcine insulin (41 mg).

The [B30-Thr($OBu^t$)-$OBu^t$]-porcine insulin obtained is converted according to example 1 (3) into human insulin; yield 63 mg.

**Claims**

1. A process for preparing a B30-threonine-insulin which comprises

(a) reacting a threonine derivative of the general formula I

$$Thr(R^1)(R^2) \qquad (I)$$

wherein Thr is the L-threonine group, $R^1$ denotes a hydrogen atom or a hydroxyl-protecting group and $R^2$ is a carboxyl-protecting group, with a des-B30-insulin in a molar ratio of 5 : 1 to 1000 : 1 in the presence of an enzyme specifically acting on the basic amino acid carbonyl groups in peptide linkages and

(b) if desired, removing the protecting group(s) from the B30-Thr($R^1$)($R^2$)-insulin obtained.

2. The process according to claim 1, wherein said enzyme is trypsin or a trypsin-like enzyme.

3. The process according to claim 2, wherein said enzyme is trypsin.

4. The process according to claim 2, wherein said trypsin-like enzyme is protease I.

5. The process according to claim 1, wherein the molar ratio is 25 : 1 to 200 : 1.

6. The process according to claim 1, wherein the reaction is effected in a medium containing one or more organic solvents.

7. The process according to claim 6, wherein the organic solvent is selected from methanol, ethanol, dimethylformamide, dimethyl sulfoxide and glycerol.

8. The process according to claim 7, wherein the organic solvent is selected from ethanol and dimethylformamide.

9. The process according to claim 1, wherein the des-B30-insulin is des-B30-porcine-insulin.

10. The process according to claim 1, wherein the des-B30-insulin is des-B30-bovine-insulin.

11. The process according to claim 1, wherein the threonine derivative is L-threonine tert.-butyl ester.

12. The process according to claim 1, wherein the reaction is effected at pH 5 to pH 9.

13. The process according to claim 12, wherein the reaction is effected at pH 6 to pH7.

14. The process according to claim 1, wherein the reaction is effected at a temperature of 0 to 50°C.

15. The process according to claim 14, wherein the reaction is effected at a temperature of 20 to 40°C.

16. A B30-Thr($R^1$)($R^2$)-insulin, wherein Thr is the L-threonine group, $R^1$ denotes a hydrogen atom or a hydroxyl-protecting group and $R^2$ is a carboxyl-protecting group.

**Revendications**

1. Procédé pour préparer une B30-thréonine-insuline qui comprend:

a) la réaction d'un dérivé de thréonine représenté par la formule I

$$Thr(R^1)(R^2) \quad (I)$$

dans laquelle Thr représente un résidu de L-thréonine, $R^1$ un atome d'hydrogène ou un groupe protecteur de l'hydroxy, et $R^2$ un groupe protecteur du carboxy, avec une des B30-insuline, dans un rapport molaire de 5 : 1 à 1000 : 1, en présence d'une enzyme agissant de façon spécifique sur les groupes carbonyle d'amino-acides basiques dans les liaisons peptidiques, et

b) si on le désire, l'élimination du (des) groupe(s) protecteur(s) de la B30-Thr($R^1$)($R^2$)-insuline obtenue.

2. Procédé selon la revendication 1, caractérisé en ce que ladite enzyme est la trypsine ou une enzyme analogue à la trypsine.

3. Procédé selon la revendication 2, caractérisé en ce que ladite enzyme est la trypsine.

4. Procédé selon la revendication 2, caractérisé en ce que ladite enzyme analogue à la trypsine est la protéase I.

5. Procédé selon la revendication 1, caractérisé en ce que la proportion molaire est de 25 : 1 à 200 : 1.

6. Procédé selon la revendication 1, caractérisé en ce que la réaction est effectuée dans un milieu contenant un ou plusieurs solvants organiques.

7. Procédé selon la revendication 6, caractérisé en ce que le solvant organique est choisi parmi le méthanol, l'éthanol, le diméthylformamide, le diméthylsulfoxyde et le glycérol.

8. Procédé selon la revendication 7, caractérisé en ce que le solvant organique est choisi parmi l'éthanol et le diméthylformamide.

9. Procédé selon la revendication 1, caractérisé en ce que la dés-B30-insuline est la dés-B30-insuline porcine.

10. Procédé selon la revendication I, caractérisé en ce que la dés-B30-insuline est la dés-B30-insuline bovine.

11. Procédé selon la revendication 1, caractérisé en ce que le dérivé de thréonine est l'ester t-butylique de la L-thréonine.

12. Procédé selon la revendication 1, caractérisé en ce que la réaction est effectuée à pH 5 à pH 9.

13. Procédé selon la revendication 12, caractérisé en ce que la réaction est effectuée à pH 6 à pH 7.

14. Procédé selon la revendication 1, caractérisé en ce que la réaction est effectuée à une température de 0 à 50°C.

15. Procédé selon la revendication 14, caractérisé en ce que la réaction est effectuée à une température de 20 à 40°C.

16. B30-Thr($R^1$)($R^2$)-insuline, dans laquelle Thr représente le groupe L-thréonine, $R^1$ un atome d'hydrogène ou un groupe protecteur d'hydroxyle et $R^2$ un groupe protecteur de carboxyle.

**Patentansprüche**

1. Verfahren zur Herstellung von B30-Threonin-insulin, dadurch gekennzeichnet, daß man

a) ein Threoninderivat der allgemeinen Formel I

$$Thr(R^1)(R^2) \quad (I)$$

in der Thr die L-Threoningruppe bedeutet, $R^1$ ein Wasserstoffatom oder eine Hydroxyl-Schutzgruppe darstellt und $R^2$ eine Carboxyl-Schutzgruppe bedeutet, mit einem des-B30-Insulin in einem Molverhältnis von 5 : 1 bis 1000 : 1 in Gegenwart eines Enzyms zur Umsetzung bringt, das spezifische Wirksamkeit gegenüber den Carbonylgruppen in den Peptidbindungen der basischen Aminosäure aufweist, und

b) gegebenenfalls die Schutzgruppe(n) aus dem erhaltenen B30-Thr($R^1$)($R^2$)-Insulin abspaltet.

2. Verfahren nach Anspruch 1, dadurch gekennzeichnet, daß man als Enzym Trypsin oder ein trypsinähnliches Enzym einsetzt.

3. Verfahren nach Anspruch 2, dadurch gekennzeichnet, daß man als Enzym Trypsin verwendet.

4. Verfahren nach Anspruch 2, dadurch gekennzeichnet, daß man als trypsinähnliches Enzym Protease I verwendet.

5. Verfahren nach Anspruch 1, dadurch gekennzeichnet, daß das Molverhältnis 25 : 1 bis 200 : 1 beträgt.

6. Verfahren nach Anspruch 1, dadurch gekennzeichnet, daß man die Umsetzung in einem Medium durchführt, das mindestens ein organisches Lösungsmittel enthält.

7. Verfahren nach Anspruch 6, dadurch gekennzeichnet, daß man als organisches Lösungsmittel Methanol, Äthanol, Dimethylformamid, Dimethylsulfoxid oder Glycerin verwendet.

8. Verfahren nach Anspruch 7, dadurch gekennzeichnet, daß man als organisches Lösungsmittel Äthanol oder Dimethylformamid einsetzt.

9. Verfahren nach Anspruch 1, dadurch gekennzeichnet, daß man als des-B30-Insulin das Schweine-des-B30-Insulin einsetzt.

10. Verfahren nach Anspruch 1, dadurch gekennzeichnet, daß man als des-B30-Insulin das Rinder-des-B30-Insulin einsetzt.

11. Verfahren nach Anspruch 1, dadurch gekennzeichnet, daß das Threoninderivat L-Threonin-tert.-butylester ist.

12. Verfahren nach Anspruch 1, dadurch gekennzeichnet, daß man die Umsetzung bei einem pH-Wert von 5 bis 9 durchführt.

13. Verfahren nach Anspruch 12, dadurch gekennzeichnet, daß man die Umsetzung bei einem pH-Wert von 6 bis 7 durchführt.

14. Verfahren nach Anspruch 1, dadurch gekennzeichnet, daß man die Umsetzung bei Temperaturen von 0 bis 50°C durchführt.

15. Verfahren nach Anspruch 14, dadurch gekennzeichnet, daß man die Umsetzung bei Temperaturen von 20 bis 40°C durchführt.

16. Ein B30-Thr($R^1$)($R^2$)-Insulin, dadurch gekennzeichnet, daß Thr den L-Threoninrest bedeutet, $R^1$ ein Wasserstoffatom oder eine Hydroxyl-Schutzgruppe darstellt und $R^2$ eine Carboxyl-Schutzgruppe bedeutet.